# EUROPEAN PATENT APPLICATION

(11) **EP 2 360 653 A2**
(43) Date of publication of application: **24.08.2011**
(21) Application number: 09826247.0
(22) Date of filing: 05.11.2009
(51) Int. Cl.: G08B 25/10, H04M 1/24, H01Q 1/00

(54) **EMERGENCY RESCUE SYSTEM USING EYE EXPRESSION RECOGNITION, AND METHOD FOR SAME**

(30) Priority: 17.11.2008 KR 20080114077
(71) Applicant: Jeon, Byong-Hoon, Seoul 137-806 (KR)
(72) Inventor: Jeon, Byong-Hoon, Seoul 137-806 (KR)
(74) Representative: Lermer, Christoph
(86) International application number: PCT/KR2009/006493
(87) International publication number: WO 2010/056015

(57) **Abstract**

Disclosed is an emergency rescue system triggered by eye expression recognition, comprising an eye expression recognition based emergency rescue client and an eye expression recognition based emergency rescue server which are connected each other over wired or wireless network, wherein said eye expression recognition based emergency rescue client composes a rescue requesting message and transfers it to the eye expression recognition based emergency rescue server, wherein the rescue requesting message is created in combining two group of rescue requesting signals and processing them with reference to a prepared message conversion database; the first signal which represents, on virtual plane, a series of patterns of a rescue requester's eye gaze change or movement derived from his(her) eye components, and the second signal which represents the change of his(her) intentional eyelid blinking; and said eye expression recognition based emergency rescue server judges the emergency situation by analyzing the received rescue requesting message, retrieves the rescue information corresponding to the emergency situation from the rescue information database, takes the system-predefined emergency rescue counter measures on the basis of the retrieval results, or reports the emergency rescue request directly to the pre-routed servers of other emergency rescue associated organizations.

## Description

### Technical Field

The present invention relates to an emergency rescue system triggered by an eye expression recognition which makes it possible to provide a safe emergency rescue request and an automatic remote monitoring on the basis of the request and a fast emergency rescue service in such a manner that a rescue message is prepared by tracking and recognizing the changes of an eye gaze detected from the eyes' components of a person who needs rescue, and the prepared rescue message is transferred to an emergency rescue server.

### Background Art

The eye gaze tracking has been increasingly applied to the various industries such as vehicle industry with the application of monitoring a driver's inattentiveness to forward roadway, or internet industry with evaluating the effect of online advertisement based on, what is called, "Passive eye monitoring" technology, and also such as the disabled friendly products like eye-mouse or eye-writing based on "Active eye monitoring" technology.

The eye gaze tracking technology can be also usefully applied for the countermeasure of requesting emergency rescue, especially, in the case that a person in danger has not any alternative except his(her)eye movement to propagate the current emergency situation to the outside. In particular, an emergency rescue message preparation based on an active eye gaze tracking which is converted into a digital messaging system, and which can be unrecognizably transferred to a remote emergency rescue center over wired or wireless communication network can be an effective measure when a person is in severely dangerous situation which does not allow to make a call or even push the emergency button because of the threatening or strict watching from other person(s) in a vehicle.

### Disclosure of Invention

Accordingly, the objective of the present invention is to provide an emergency rescue system based on the eye expression recognition which enables to request an emergency rescue to a remote place over wired or wireless network and to get a proper rescue service when a person in emergency situation is unable to take any action except his(her) eye movement. To achieve the above objective, there is provided emergency rescue system triggered by eye expression recognition, comprising an eye expression recognition based emergency rescue client and an eye expression recognition based emergency rescue server which are connected each other over wired or wireless network, wherein said eye expression recognition based emergency rescue client composes a rescue requesting message and transfers it to the eye expression recognition based emergency rescue server, wherein the rescue requesting message is created in combining two group of rescue requesting signals and processing them with reference to a prepared message conversion database; the first signal which represents, on virtual plane, a series of patterns of a rescue requester's eye gaze change or movement derived from his(her) eye components, and the second signal which represents the change of his(her) intentional eyelid blinking; and said eye expression recognition based emergency rescue server judges the emergency situation by analyzing the received rescue requesting message, retrieves the rescue information corresponding to the emergency situation from the rescue information database, takes the system-predefined emergency rescue measures on the basis of the retrieval results, or reports the emergency rescue request to the pre-routed servers of other emergency rescue associated organizations.

Preferably, the eye expression recognition based emergency rescue client transfers its position information or identification information to the eye expression recognition based emergency rescue server along with a rescue requesting message.

To achieve the above objective, there is provided emergency rescue method triggered by eye expression recognition, wherein said emergency rescue method is adapted to an emergency rescue system triggered by eye expression which consists of an eye expression recognition based emergency rescue client and an eye expression recognition based emergency rescue server which are connected each other over wired or wireless network, comprising a step in which the eye expression recognition based emergency rescue client monitors the change of eye gaze and intentional eyelid blinking derived from the eye components of a rescue requester through security camera(s); a step in which the eye expression recognition based emergency rescue client composes a rescue requesting signal in a combination with the signal representing a series of patterns by tracking the rescue requester's eye gaze change as the first type which is displayed on a virtual 2D or 3D plane and the signal of his (or her) intentional eyelid blinking as the second, and thereafter converts it into a rescue requesting message with reference to the message conversion database no matter when the said client recognizes that the rescue requester begins to communicate or request an emergency rescue by changing his(or her) eye gaze or the number of times of eyelid blinking; a step in which the eye expression recognition based emergency rescue client transfers the converted rescue requesting message with the real-time position information to the eye expression recognition based emergency rescue server over wired or wireless network; a step in which the eye expression recognition based emergency rescue server judges the emergency situation by analyzing the received rescue requesting message and retrieves a rescue information corresponding to the emergency situation with the help of the rescue information database; a step in which the eye expression recognition based emergency rescue server identifies which eye expression recognition based emergency rescue client is under an emergency situation by the received identification information with the help of customer information database, and tracking, in real time, the geographical location of the eye expression recognition based emergency rescue client with the help of geographic information system database in use of the received geographical position information; and a step in which the eye expression recognition based emergency rescue server takes the system-predefined emergency rescue measures on the basis of the retrieved rescue information and the found geographical location when the eye expression recognition based emergency rescue client under emergency situation is approved as an authorized service subscriber in checking the received identification information, and furthermore transfers the emergency rescue requesting information to the pre-routed servers of other emergency rescue associated organizations even when the eye expression recognition based emergency rescue client is not an authorized service subscriber.

### Effects

The present invention has the distinctive advantage comparing to the legacy emergency rescue systems, in everyday life of the circumstances in which a rescue requester can be frequently exposed to the physical threatening by the deadly weapon such as a knife or gun, in that it enables to propagate a emergency rescue request in a manner of being unexposed to the threatening person(s) even when he(she) confronts with the imminently dangerous situation not allowing any possible chance of the emergency rescue request by voice or any tiny gesture due to the extremely intensive watching.

### Brief Description of the Drawings

The present invention will become better understood with reference to the accompanying drawings which are given only by way of illustration and thus are not limitative of the present invention, wherein;
Figure 1 is an illustration of the network configuration of an emergency rescue system triggered by eye expression recognition according to the present invention;
Figure 2 is a functional block diagram of eye expression recognition based emergency rescue client 100 and 200 according to the present invention;
Figure 3 is a functional block diagram of an eye expression recognition based emergency rescue server 300 according to the present invention; and
Figure 4 is an illustration of the recognition of a rescue signal of by eye expression recognition detection module according to the present invention.

### Modes for carrying out the invention

The preferred embodiments of the present invention will be described with reference to the accompanying drawings.

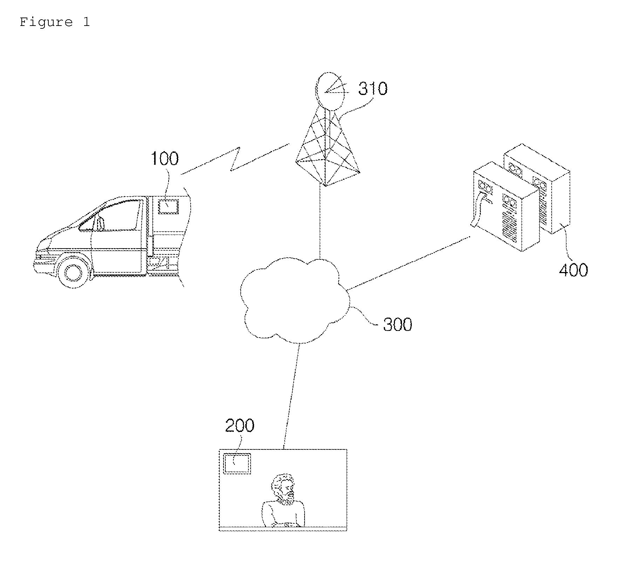

Figure 1 is an illustration of the network configuration of an emergency rescue system triggered by eye expression recognition according to the present invention.

The term "eye expression recognition" used in the descriptions of the present invention means recognizing a rescue requesting signal including all possible communication contents formed in a combination with two groups of signals; main signal (character, text, symbol, code or geometrical graphic drawing) formed of line, arc and circle expressed on a virtual 2D or 3D plane created by measuring the change of eye gaze such as gaze angle, direction or movement derived from eye components such as pupils, irises, eyelids, eyeballs and etc., and an auxiliary signal generated due to the intentional change (number of times, duration, state of eye closure) of eye blinking.

As shown in the drawings, an eye expression recognition based emergency rescue client 100, 200 installed inside a vehicle or a facility such as a convenience store or a bank is connected with an eye expression recognition based emergency rescue server 400 so that they communicate each other bi-directionally over wired or wireless network 310 and 320. The eye expression recognition based emergency rescue server 400 functions as a main control center in response to the multiple eye expression recognition based emergency rescue clients 100 and 200.

Here, the wired network includes a conventional wired internet, and the wireless network includes a mobile internet network, a mobile communication network or a local area wireless data communication network.

According to an aspect of the present invention, the eye expression recognition based emergency rescue client 100, 200 composes a rescue requesting message which is created in combining two group of rescue requesting signals and processing them with reference to a prepared message conversion database; the first signal which represents, on virtual plane, a series of patterns of a rescue requester's eye gaze change or movement derived from his(her) eye components, and the second signal which represents the change of his(her) intentional eyelid blinking and then the eye expression recognition based emergency rescue client 100, 200 transfers it to the eye expression recognition based emergency rescue server 400 over the wired or wireless network 300, 310 along with an identification information. Here, the rescue message from the eye expression recognition based emergency rescue client 100, 200 might include the visual information of a rescue requester.

As for another aspect of the present invention, the eye expression recognition based emergency rescue client 100, 200 provides a table of emergency rescue signals on a virtual spatial display, converts the information selected from the table in conjunction with a rescue requester's eye gaze change and eyelid blinking into rescue requesting messages with reference to a prepared message conversion database and transfers them to the eye expression recognition based emergency rescue server 400 over wired or wireless network 300, 310. In other words, once a rescue requester composes a rescue requesting message by his(her) eye gaze change or eye blinking with the provided table of emergency rescue signals, the eye expression recognition based emergency rescue client 100, 200 converts it into a rescue message with reference to the message conversion database.

In addition, the eye expression recognition based emergency rescue server 400 judges the emergency situation by analyzing the received rescue requesting message, and retrieves the rescue information corresponding to the emergency situation from the rescue information database, and takes the system-predefined emergency rescue measures on the basis of a result of the retrieval or reports the emergency rescue request to the pre-routed servers of other emergency rescue associated organizations. Preferably, the eye expression recognition based emergency rescue server 400 starts the remote real-time visual monitoring and secured emergency communication in stealth way in order that it can quickly judge the emergency situation and take additional appropriate safety measures on receiving a rescue requesting message, and then provides the counter measuring instructions for the eye expression recognition based emergency rescue client 100, 200.

According to the above-described construction of the present invention, in everyday life of the circumstances in which a rescue requester can be frequently exposed to the physical threatening by the deadly weapon such as a knife or gun, it enables to propagate a emergency rescue request in a manner of being unexposed to the threatening person(s) even when he(she) confronts with the imminently dangerous situation not allowing any possible chance of the emergency rescue request by voice or any tiny gesture due to the extremely intensive watching.

The emergency rescue system of the present invention can be applied for a wide range of applications. The present invention can be applicable for the protection of vehicle driver from an emergency situation such as threatening, kidnapping or robbing by fellow passenger (in side seat or rear seat) holding a knife or a gun in the transportation vehicles such as car, especially taxi or cash transportation vehicle.

In addition, the emergency rescue system of the present invention can be applicable for the following areas: notifying in a undisclosed manner the compelled unlocking of secured facilities due to a certain physical threatening or the attempts of mugging and robbery in a bank, grocery store or building or elsewhere; reporting the crime-wanted in a vehicle; emergency communication instrument of requesting emergency rescue; writing and transferring a emergency rescue requesting message in SMS form while at the wheel; instructing or manipulating vehicle mounted devices(telematics or other electronic devices) while driving.

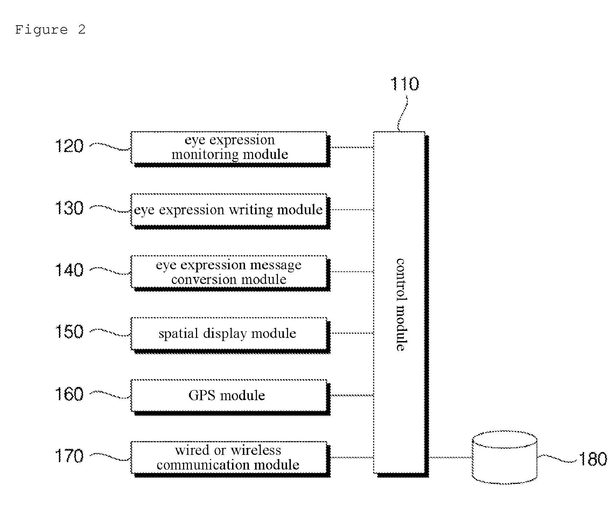

Figure 2 is a functional block diagram of an eye expression recognition based emergency rescue client 100, 200 according to the present invention.

The eye expression monitoring module 120 monitors the change of eye gaze and intentional change of eyelid blinking derived from a rescue requester's eye components. In detail, the eye expression recognition monitoring module 120 is formed of day and night security camera(s) which is capable of day and night monitoring, focusing on eye pupils of a rescue requester such as a driver in a vehicle or a bank teller at a bank or other place. Here, preferably, the day and night security camera might be controlled for pan, tilt, or zoom function by a remote server.

Here, the recognition process for an intentional message or an emergency rescue request from a rescue requester is triggered by the combination with the change of eye gaze or the number of blinking during a given preset time duration.

The eye expression writing module 130 composes a rescue requesting signal including all possible communication contents intended to transfer in combination with two groups of signals; main signal (character, text, symbol, code or geometrical graphic drawing) formed of line, arc and circle expressed on a virtual 2D or 3D plane created by measuring the change of eye gaze such as gaze angle, direction or movement derived from eye components such as pupils, irises, eyelids, eyeballs and etc., and an auxiliary signal generated due to the intentional change(number of times, duration, state of eye closure) of eye blinking.

In other words, the eye expression writing module 130 has the functions of composing an intended message or emergency rescue signal of a rescue requester and the function of displaying characters after the process of mapping or combining with character, text, symbol, code, or geometric graphic drawing, on virtual spatial display, created according to the change of eye gaze. Furthermore, the eye expression writing module 130 provides the editing, making out, or other functions necessary for writing main signal as it assigns eye-blink as an auxiliary function key while tracking the sequentially caught multiple eye-blink keys within a given preset time duration. For instance, there can be used "eye-blink + eye-blink + eye-direction key + eye-blink" for editing the previously written characters. At this time, the eye-direction key represents the line trace which eye gaze creates moving consistently to a certain direction of up/down or left/right, for example. The eye expression writing module 130 may use eye-blink key for projecting an emergency rescue signal table on a virtual spatial display, which the rescue requester frequently or urgently uses, in order to enable him(her) to promptly and conveniently get works done.

The rescue requester can select a signal with the projected emergency rescue signal table using eye-direction keys in left, right, up or down gaze direction and the eye-blink key. As another auxiliary key, there is eye-focus key for fixing the eye focus (fixation) on a specific position within a given preset time duration.

The eye expression message conversion module 140 converts a rescue requesting signal created by the eye expression writing module 130 into a rescue requesting message with reference to the message conversion database 180. Here, the rescue requesting message represents a digital character (s)or text corresponding to the rescue requesting signal.

In interaction with the eye expression writing module 130, the virtual spatial display module 150 has the function of projecting and displaying, not on any physical surface but on a virtual spatial surface in order to avoid being realized by a threatening person, what the rescue requester creates as for the emergency rescue requesting message and signal, and displays an externally incoming message on the spatial display as well. Preferably, the said spatial display module comprises a display which has the property of controlled field of view in order to keep the message from being disclosed.

The GPS module 160 receives the information of the current position of the eye expression recognition based emergency rescue client 100, 200 from a GPS satellite and transfers it to the eye expression recognition based emergency rescue server 400 along with the rescue requesting message corresponding to the composed rescue signal. Here, when the eye expression recognition based emergency rescue client is installed at a certain fixed facility, the GPS module 160 is not needed. Consequently, it is needed to send only the identification information of the eye expression recognition based emergency rescue client.

The wired or wireless communication module 170 is a bidirectional communication module which can transfer the rescue requesting message of the rescue requester to the eye expression recognition based emergency rescue server 400 and receive the emergency countermeasure information from the eye expression recognition based emergency rescue server 400. Here, the wireless communication module represents all kinds of wired or wireless communication modules, comprising mobile internet communication function such as Wibro (so called Mobile WiMax), HSDPA/HSUPA, GPRS, TETRA, or others, and mobile communication network function such as WCDMA, CDMA/GSM, TD-SCDMA, UMB, LTE, or other type of mobile communication and other short range wireless data communication function such as WiFi, DSRC, Bluetooth, etc.

The control module 110 controls each module 120, 130, 140, 150, 160 and 170 and has a function of controlling and managing the inputs and outputs to and from the message conversion database 180.

The message conversion database 180 is the predefined interpretation database which translates an emergency rescue request into an emergency rescue requesting message, which the request is either the combination of main signal(s) which the eye expression writing module 130 composes and eye-blink key as an auxiliary signal or an emergency rescue request which a rescue requester composes in selecting a signal with the emergency rescue signal table.

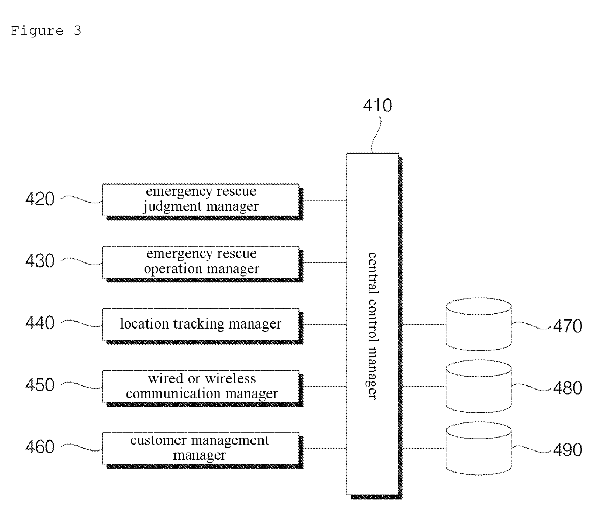

Figure 3 is a functional block diagram of an eye expression recognition based emergency rescue server 300 according to the present invention.

The emergency rescue judgment manager 420 analyzes the rescue requesting message from the eye expression recognition based emergency rescue client 100, 200, judges the emergency situation and retrieves rescue information corresponding to the emergency situation with the help of the rescue information database 490.

The location tracking manager 440 tracks the location information of the eye expression recognition based emergency rescue client 100, 200 from both the customer information database 470 and the geographic information system database 480 on the basis of the received position information from the eye expression recognition based emergency rescue client 100, 200.

The emergency rescue operation manager 430 is directed to systematically performing the emergency rescue countermeasure on the basis of the retrieved rescue information and the tracked location information.

The customer management manager 460 is directed to maintaining both the customer information of the emergency rescue requester and the profile information of the eye expression recognition based emergency rescue client 100, 200.

The wired or wireless communication manager 450 controls and manages a bidirectional real time communication with the eye expression recognition based emergency rescue client 100, 200 and checks over the status of non-interrupted communication with the pre-routed external servers of emergency rescue associated organizations.

The central control manager 410 is directed to controlling the operations of each manager 420, 430, 440, 450 and 460 and the inputs and outputs to and from each database 470, 480 and 490. Here, the customer information database 470 maintains the information related to the subscribed customer, and the geographic information system database 480 maintains geographical map information for real time location tracking, and the rescue information database 490 maintains all the information related to emergency rescue counter measures.

The operations of the eye expression recognition based emergency rescue system according to the present invention with the above-described constructions will be described.

For facilitating to describe, it is assumed that the eye expression recognition based emergency rescue client 100 is installed inside a vehicle, and the driver of the vehicle is under threat from a certain person.

The eye expression monitoring module120 installed inside the vehicle monitors the change of eye gaze such as gaze angle, direction, or movement derived from eye components such as pupils, irises, eyelids, and eyeballs together with the driver's intentional change of eyelid blinking. At this time, it is recognized as the driver's intention to communicate or request a emergency rescue when the change of eye gaze in combination with the number of times of eye blinking is detected within a given preset time duration, and the eye expression monitoring module 120 comes to invoke the eye expression writing module 130 by requesting to run it to the control module 110.

The eye expression writing module 130 composes a rescue requesting signal indicating the driver's intent to be transferred in combination with two groups of signals; main signal (character, text, symbol, code or geometrical graphic drawing) formed of line, arc and circle expressed on a virtual 2D or 3D plane created by measuring the change of eye gaze such as eye angle, direction or movement derived from eye components such as pupils, irises, eyelids, eyeballs and etc., and an auxiliary signal generated due to the intentional change(number of times, duration, state of eye closure)of eye blinking.

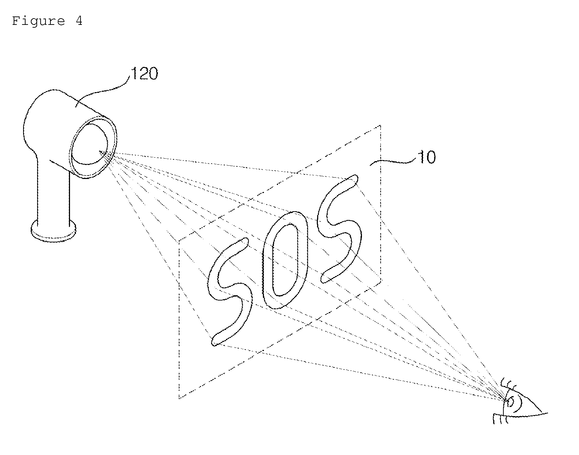

As shown in Figure 4, the eye expression writing module 130 displays, on the virtual display 10 of spatial projection provided by the virtual spatial display module 150, the mapped or combined characters with character, text, symbol, code, or geometric graphic drawing which the change of eye gaze makes based on eye components, and at the same time, the eye expression monitoring module 120 connected with the monitoring camera recognizes those characters.

The eye expression message conversion module 140 is directed to converting the rescue signal created by the eye expression writing module 130 into a rescue requesting message with reference to the message conversion database 180.

The GPS module 160 receives in real time the information related to the current position of the eye expression recognition based emergency rescue client 100, 200 and transfers it to the control module 110.

The control module 110 transfers the rescue requesting message converted by the eye expression conversion module 140 and the position information provided from the GPS module 160 to the eye expression based emergency rescue server 400 over the wired or wireless communication module 170. At the same time, the control module 110 transfers the identification information of the eye expression recognition based emergency rescue client 100 as well so that the eye expression recognition based emergency rescue server 400 can identify the eye expression recognition based emergency rescue client 100 under the emergency situation.

The emergency rescue judgment manager 420 out of the eye expression recognition based emergency rescue server 400 judges the emergency situation by analyzing the rescue requesting message received from the eye expression recognition based emergency rescue client 100, 200 installed inside a vehicle, and retrieves the rescue information corresponding to a corresponding emergency situation from the rescue information database 490.

The location tracking manager 440 identifies the eye expression recognition based emergency rescue client 100 under an emergency rescue situation with the help of the customer information database 470 on the basis of the identification information received from the eye expression recognition based emergency rescue client 100, and tracks in real time the geographical location of the eye expression recognition based emergency rescue client 100 with the help of the geographic information system database 480 on the basis of the received position information.

The emergency rescue operation manager 430 takes the system-predefined emergency rescue measures on the basis of the retrieved rescue information and the tracked geographical location when the eye expression based emergency rescue client 100 is an authorized service subscriber on the received identification information basis. For example, the emergency rescue request information can be transferred to the in-house server in operation or other interconnected servers of emergency rescue related service firms.

On the other hand, in case that the eye expression recognition based emergency rescue client 100 is proved as an unauthorized subscriber by the eye expression recognition based emergency rescue server 400 or is unable to communicate with the eye expression recognition based emergency rescue server 400, the eye expression recognition based emergency rescue client 100 transfers an emergency rescue request information directly to the pre-routed servers of other emergency rescue associated organizations such as police office or 911 center over wired or wireless network.

In case that only short range wireless data communication network is available, for example, because of network problem, it is possible that the short range wireless data device installed at a highway tollgate such as an automated fare collection system can automatically receive the rescue request signals by changing the device appropriately .

As the present invention may be embodied in several forms without departing from the spirit or essential characteristics thereof, it should also be understood that the above-described examples are not limited by any of the details of the foregoing description, unless otherwise specified, but rather should be construed broadly within its spirit and scope as defined in the appended claims, and therefore all changes and modifications that fall within the meets and bounds of the claims, or equivalences of such meets and bounds are therefore intended to be embraced by the appended claims.

## Claims

1. An emergency rescue system triggered by eye expression recognition, comprising:
an eye expression recognition based emergency rescue client and an eye expression recognition based emergency rescue server which are connected each other over wired or wireless network,
wherein said eye expression recognition based emergency rescue client composes a rescue requesting message and transfers it to the eye expression recognition based emergency rescue server,
wherein the rescue requesting message is created by combining two groups of rescue requesting signals and processing them with reference to a prepared message conversion database; the first signal which represents, on virtual plane, a series of patterns of a rescue requester's eye gaze change or movement derived from his(her) eye components, and the second signal which represents the change of his(her) intentional eyelid blinking; and
said eye expression recognition based emergency rescue server judges the emergency situation by analyzing the received rescue requesting message, retrieves the rescue information corresponding to the emergency situation from a rescue information database, takes the system-predefined emergency rescue measures on the basis of the retrieval results, or reports the emergency rescue request to the pre-routed servers of other emergency rescue associated organizations.

2. An emergency rescue system triggered by eye expression recognition, comprising:
an eye expression recognition based emergency rescue client and an eye expression recognition based emergency rescue server which are connected each other over wired or wireless network,
wherein said eye expression recognition based emergency rescue client provides a table of emergency rescue signals on a virtual spatial display, converts the information selected from the table in conjunction with a rescue requester's eye gaze change and eyelid blinking into rescue requesting messages with reference to a prepared message conversion database and transfers them to the eye expression recognition based emergency rescue server over wired or wireless network; and
said eye expression recognition based emergency rescue server judges the emergency situation by analyzing the received rescue requesting message, retrieves the rescue information corresponding to the emergency situation from the rescue information database, takes the system-predefined emergency rescue measures on the basis the retrieval results, or reports the emergency rescue request to the pre-routed servers of other emergency rescue associated organizations.

3. The system of either claim 1 or claim 2, wherein when said eye expression recognition based emergency rescue client is not an authorized subscriber on the eye expression recognition based emergency rescue server, or unable to transfer a rescue requesting message to the eye expression recognition based emergency rescue server, the eye expression recognition based emergency rescue client reports directly to the pre-routed servers of other emergency rescue associated organizations over wired or wireless network.

4. The system of either claim 1 or claim 2, wherein said eye expression recognition based emergency rescue client transfers the said rescue requesting message along with the visual information of which the rescue requester confronts the emergency situation

5. The system of claim 1, wherein said eye expression recognition based emergency rescue client transfers its position information or identification information to the eye expression recognition based emergency rescue server along with a rescue requesting message.

6. An emergency rescue system triggered by eye expression recognition, comprising:
an eye expression recognition based emergency rescue client and an eye expression recognition based emergency rescue server which are connected each other over wired or wireless network,
wherein said eye expression recognition based emergency rescue client comprises:
an eye expression monitoring module which monitors the change of eye gaze and intentional change of eyelid blinking derived from a rescue requester's eye components;
an eye expression writing module which composes a rescue requesting signal in a combination with a series of patterns displayed on a virtual plane on the basis of eye gaze change provided from the eye expression monitoring module and an intentional change of eyelid blinking;
an eye expression message conversion module which converts the rescue requesting signal created by the eye expression writing module into a rescue requesting message with reference to the message conversion database; and
a control module which controls the operation of the modules and transfers the converted rescue requesting messages along with the identification information of the eye expression recognition based emergency rescue client to the eye expression recognition based emergency rescue server through the wired or wireless communication module, and
said eye expression recognition based emergency rescue server comprises:
an emergency rescue judgment module which analyzes the rescue requesting message from the eye expression recognition based emergency rescue client, judges the emergency situation, and retrieves a rescue information corresponding to the emergency situation with the help of the rescue information database;
a location tracking module which finds the location information of the eye expression recognition based emergency rescue client by the said identification information matching with the customer information database;
an emergency rescue operation module which takes the system-predefined emergency rescue measures on the basis of the retrieved rescue information and the found location information; and
a central control module which controls the operations of the modules and database input and output.

7. The system of claim 6, wherein said eye expression recognition based emergency rescue client further comprises a GPS module for receiving real- time position information from GPS satellites, and said control module transfers the real-time position information in cooperation with the GPS module along with the rescue requesting message to the eye expression recognition based emergency rescue server, and said location tracking module of the eye expression recognition based emergency rescue server extracts a geographical location information from the geographic information system database on the basis of the received real-time position information.

8. The system of claim 6, wherein said eye expression recognition based emergency rescue client further comprises a virtual spatial display module which interacts with the eye expression writing module, projects the emergency rescue requesting message and signal which the rescue requester creates and displays an externally incoming message on the spatial display, and said spatial display module comprises a display which has the property of controlled field of view in order to keep the message from being disclosed.

9. An emergency rescue method triggered by eye expression recognition, wherein said emergency rescue method is adapted to an emergency rescue system triggered by eye expression which consists of an eye expression recognition based emergency rescue client and an eye expression recognition based emergency rescue server which are connected each other over wired or wireless network, comprising:
a step in which the eye expression recognition based emergency rescue client monitors the change of eye gaze and intentional eyelid blinking derived from the eye components of a rescue requester through security camera(s);
a step in which the eye expression recognition based emergency rescue client composes a rescue requesting signal in combination with the signal representing a series of patterns by tracking the rescue requester's eye gaze change as the first type which is displayed on a virtual 2D or 3D plane and the signal of his(or her) intentional eyelid blinking as the second, and thereafter converts it into a rescue requesting message with reference to the message conversion database no matter when the said client recognizes that the rescue requester begins to communicate or request an emergency rescue by changing his(or her) eye gaze or the number of times of eyelid blinking;
a step in which the eye expression recognition based emergency rescue client transfers the converted rescue requesting message with the real-time position information to the eye expression recognition based emergency rescue server over wired or wireless network;
a step in which the eye expression recognition based emergency rescue server judges the emergency situation by analyzing the received rescue requesting message and retrieves a rescue information corresponding to the emergency situation with the help of the rescue information database;
a step in which the eye expression recognition based emergency rescue server identifies which eye expression recognition based emergency rescue client is under emergency situation by the received identification information with the help of customer information database, and tracking, in real time, the geographical location of the eye expression recognition based emergency rescue client with the help of geographic information system database in use of the received geographical position information; and
a step in which the eye expression recognition based emergency rescue server takes the system-predefined emergency rescue measures on the basis of the retrieved rescue information and the found geographical location when the eye expression recognition based emergency rescue client under emergency situation is approved as an authorized service subscriber in checking the received identification information, and furthermore transfers the emergency rescue requesting information to the pre-routed servers of other emergency rescue associated organizations even when the eye expression recognition based emergency rescue client is not an authorized service subscriber.
